# EUROPEAN PATENT SPECIFICATION

(11) **EP 0 479 935 B1**
(45) Date of publication and mention of the grant of the patent: **16.03.1994**
(21) Application number: 90917827.9
(22) Date of filing: 27.06.1990
(51) Int. Cl.: A61F 5/455, A61M 25/00

(54) **IMPROVEMENTS IN AND RELATING TO INCONTINENCE SYSTEMS**
VERBESSERUNGEN AN HARNENTZUGSSYSTEMEN
AMELIORATIONS RELATIVES A DES SYSTEMES DE DRAINAGE EN CAS D'INCONTINENCE

(30) Priority: 28.06.1989 SE 8902333
(43) Date of publication of application: 15.04.1992
(73) Proprietor: Nilsson, Leif, S-260 40 Viken (SE)
(72) Inventor: Nilsson, Leif, S-260 40 Viken (SE)
(74) Representative: Andersson, Andy Rudy
(86) International application number: SE9000458
(87) International publication number: WO9100074

(56) References cited:
- WO-A-82/00767
- FR-A-20 237 63
- FR-A-58 601 8
- SE-B- 380 176
- SE-B- 386 365
- US-A- 3 721 243

## Description

The present invention relates generally to an improvement in urine incontinence systems.

More specifically, but not exclusively, the invention relates to apparatus operative to remove or assist in the discharge of urine from the bladders of female patients, either periodically or continuously. Such apparatus will include a urine catheter in the form of an elongated, hollow tubular element, referred to here as a catheter tube, which is intended for insertion into the urethra or urinary tract of a female patient. One open end of the tube is in communication with the bladder and therewith functions as an inlet opening for urine present in the bladder. The other open end of the catheter tube functions as an outlet opening through which urine passes from the bladder. The catheter tube includes means by which the tube is fixated within the urethra or urinary tract. Such apparatus will also include a container for collecting and storing temporarily the urine that drains from the bladder. The container is connected to the outlet opening of the catheter tube, either directly or indirectly, and is provided in the region of said connection with valve means which allows urine to flow in one direction from the catheter tube into the container, but which is operative to prevent the flow of urine in the reverse direction. The lower end of the container incorporates means by which the container can be emptied.

Apparatus of the aforesaid kind which are intended for the removal of body fluids, particularly urine, and which thus include a drainage tube and a container for the temporary storage of body fluid that drains off are well documented in patent literature and are well known from the practical use of such apparatus in medical care establishments. The present invention thus relates to a catheter tube for internal use, as distinct from commercially available urinals intended for external use. An example of this latter type of catheter is described and illustrated in US-A-3,374,790.

Examples of catheters for the removal or drainage of urine or other body fluids and intended for internal use are found described and illustrated in U.S. Patent Specification Nos. 4,227,533; 3,583,404; 3,595,241 and 3,314,430.

Storage containers for fluids excreted from the body are known from a plurality of patent publications. See, for instance, US-A-3,568,218; DE-OS-26 34 071; GB-A-2.061.103; US-A-3,432,865 and US-A-4,270,213.

An apparatus operative to drain and collect body fluids, preferably urine, is obtained through the combination of a known catheter tube and a known container. As far as is known, medical care establishments have hitherto used this combination technique.

In addition to the known technique described above, the patent literature also teaches portable urine drainage systems or apparatus (see, for instance, US-A-3,703,-731) intended solely for use by vehicle-borne persons who may find it difficult to leave the vehicle to urinate in a conventional fashion.

However, there is a progressively increasing number of people who suffer from incontinence with accompanying difficulties and unpleasantness in the form of uncomfortable skin irritation and the increased need of personal hygiene, this latter including both body hygiene and a frequent change of clothing and bed linen.

When using conventional internal catheters, it is necessary normally for the catheters to be inserted and removed by nursing personnel. When a known urine catheter is combined with a known container, the container may be carried, for instance, on one side of the body of the person suffering from incontinence, such that urine discharged or draining from the patient's bladder will run through the catheter and into the container. Urine incontinence, however, is normally the result of weak muscles in the walls of the urethra, these muscles, which have a valve function, having been weakened by surgical operation or at birth or through some other cause. Incontinence can be reduced or alleviated by a successive build-up of the muscle in the urethra wall. This remedy requires exercising the muscles involved, however.

It will be understood that when a person suffering from incontinence is fitted with urine removal apparatus of the aforesaid kind with which urine flows continuously from the bladder into the container, the bladder will never be filled, i.e. the bladder is almost always empty, and the aforesaid muscles can never be properly exercised.

Another drawback with apparatus with which urine discharges continuously from the bladder is that the conventional containers are dimensioned to accommodate a relatively small volume of urine, say about 0.2-0.3 l, and are made of a flexible material. When filled or partly filled with urine, such a container becomes bulky and relatively extended and when supported along the leg of the user is highly troublesome and uncomfortable.

A specific object of the present invention is to provide an improved apparatus or system of the kind described in the introduction which will avoid the aforesaid drawbacks with respect to catheter appliances intended for internal use by women, particularly women who suffer from incontinence.

The improved system is characterized in that the means by which the catheter tube is fixated in the urethra includes a first non-rigid, compliant collar which is located on the outer surface of the tube, concentrically with the longitudinal axis thereof, in the region of the tube opening that coacts with the bladder, and which is intended to sealingly abut the inner wall of the urethra, and further includes a second non-rigid, compliant collar which is located adjacent to or near the outlet opening of the catheter tube, such as to form a restriction which limits the extent to which the tube can be inserted into the urethra; and is further characterized in that the urine collecting container is divided into mutually communication sections or compartments.

Firstly, the length of the improved catheter tube is adapted accurately to the length dimension of the female urethra and the tube can be inserted into and removed from the urethra with comparative ease. The rearwardly located collar also functions as a means for indicating to the user that the catheter tube has been inserted to an extent at which said collar lies in sealing abutment with the body in the region of the urethra orifice. At the same time, the collar which is located nearest the inlet opening of the catheter tube will be oriented such that the peripheral surface of the collar abuts urethra musculature and therewith ensures that an effective seal is obtained. The cross-sectional dimensions of the catheter tube are uniform overall and the tube has an outer diameter which is only slightly smaller than the diameter of the urinary tract or urethra.

Secondly, the indirect connection of the catheter tube to the urine collecting container or to the drainage hose, i.e. the hose which connects the catheter tube to the container, is provided with known, manually operable means for blocking the urine passageway and therewith prevent the flow of urine into the container. This enables urine to build up in the bladder, so that the muscles of the urethra or urinal tract will be subjected to the muscle-strenghening exercises required for remedying incontinence. It will be understood that when the catheter tube is connected directly to the container, similar blocking means can be provided on the catheter tube, e.g. in the region of the catheter opening located externally of the body of the wearer.

Thirdly, the container intended for the temporary storage of urine is composed of compartments which function to achieve uniform distribution of the urine entering the container. Despite its flexibility, the container will retain an optimal flat configuration when filled, therewith causing less trouble and discomfort to the wearer than known containers intended for this purpose.

The thus improved apparatus or system has a surprisingly pronounced psychological effect on the user, while, at the same time, automatically stimulating weakened urinal musculature.

The provision of manually operable means for closing the drainage hose (or catheter tube) enables the user to interrupt or commence emptying of the bladder at will.

Advantagous embodiments and further developments of the inventive apparatus are defined in the depending Claims.

The invention will now be described in more detail with reference to an exemplifying embodiment of the inventive apparatus or system illustrated in the accompanying schematic drawing. Other features of the improved urine drainage apparatus or system will also be apparent from the following description.

### In the accompany drawing,

Figure 1 illustrates schematically and in axial section a catheter tube for internal use; Figure 2 illustrates schematically the tube of Figure 1 connected detachably to a drainage hose;
Figure 2a illustrates schematically exemplifying means for preventing urine from flowing into a urine collecting container;
Figure 3 illustrates from above part of the drainage hose of Figure 2 or Figure 2a detachably connected to a urine collecting container; and
Figure 4 is a sectional view taken in the direction of the arrow IV-IV.

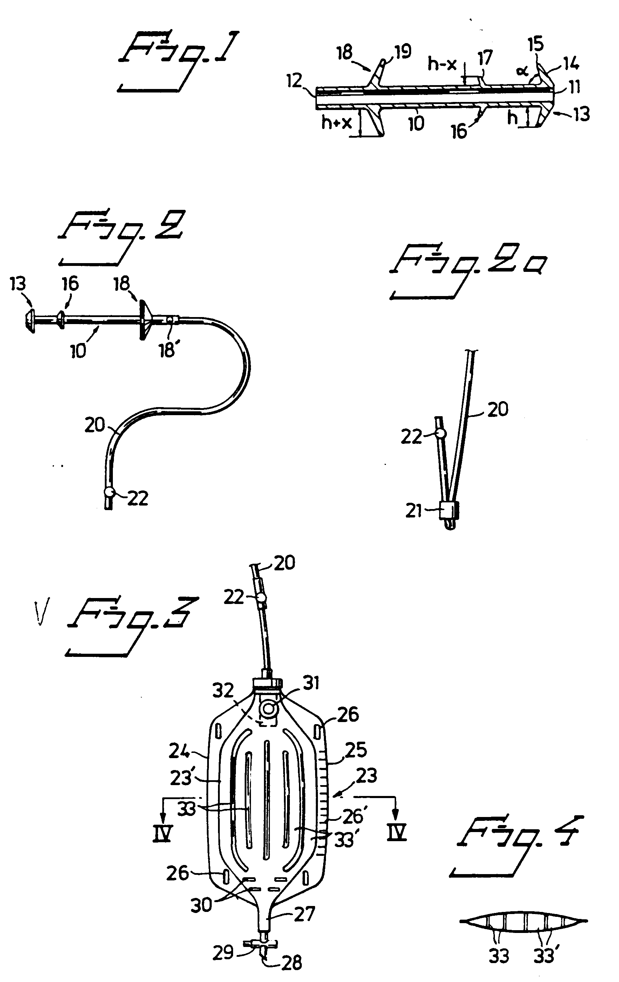

The length of the female urethra is normally about 4 cm. The catheter tube 10 is of flexible or bendable construction and has a total length which is greater than these 4 cm. The catheter tube 10 will preferably have a length of about 5-7 cm, or longer. The outer diameter of the tube 10 will, of course, be slightly smaller than the diameter of the female ure-thra and the outer surface of the tube will have optimal smoothness. The tube 10 has an inner diameter which is uniform throughout the whole length of the tube and presents a first opening 11, the inlet opening, and a second opening 12, the outlet opening. The tube 10 is provided adjacent the first opening 11, or in the region of said first opening 11, with a collar 13 in the form of a truncated cone and, comprising a cylindrical peripheral part 14 and a part 15 which tapers towards the ends of the conical part 14. As will be seen from Figure 1 and Figure 2, the conical part 14 of the collar slopes rearwardly towards the outlet opening 12 of the catheter tube 10, such as to form an acute angle, optionally an angle of between 50 and 75°, between the peripheral surface of the catheter tube 10 and the base of the conical part 14. In the illustrated embodiment, the collar 13 borders essentially on the inlet opening 11, although said collar may alternatively be positioned somewhat further down on the tube 10, i.e. slightly nearer the outlet opening 12. The collar 13 may, advantageously, be made of silicone rubber or some other material equally as friendly to the body of the wearer, since this collar is intended to be material located in the urethra of the female wearer. The conical part 14 of the collar 13 is elastically deformable, and the end of the tapering part 15 is rounded-off.

In accordance with one embodiment of the invention, the conical part 14 of the collar 13 may be hollow and accommodate a viscous fluid, for instance a hormone preparation, in which case the walls of said conical part 14 will consist of a material that is permeable to the fluid in question. With each movement made by the wearer, the conical part 14, which when the catheter tube 10 is inserted lies against the musculature of the urethra, will be subjected to bending or deformation forces which result in the creation of an overpressure within the hollow space of the conical part 14, whereby the viscous fluid enclosed therein will be forced out through the permeable wall of the conical part 14 and absorbed by the body of the wearer. The conical part 14 of the collar 13 will normally have a radial extension h of about 6 mm, measured from the periphery of the catheter tube.

The aforementioned viscous fluid can be introduced into the conical part 14 during industrial manufacture of the catheter tube 10.

As illustrated in Figures 1 and 2, the catheter tube 10 includes a third collar 16 which is located downstream of the collar 13 and the construction of which is essentially the same as the construction of the first collar 13 and has a radially extending, elastically deformable conical part 17. The vertical extension h-x of the third collar 16, however, is smaller or much smaller than the vertical extension h of the conical part 14 of the first collar 13.
The positions of the two aforedescribed collars 13 and 16 are such that respective conical parts 14, 17 will abut muscular parts of the wall of the female urethra.

The catheter tube 10 also has a second collar 18 located in the region of the outlet opening 12, preferably slightly upstream of said outlet opening. The circular-conical part 19 of the second collar 18 is essentially of uniform thickness and is preferably of solid construction. The collar 18, or conical part 19, has a vertical extension h + x, i.e. a vertical or radial extension which is greater than the vertical or radial extension of the first collar 13. The conical part 19 widens in the upstream direction of the catheter tube 10, and by making the conical part 19 compliant, said part can be brought into abutment with the urethra or urinal tract in the vicinity of the outlet orifice thereof. The conical part 19 of the second collar 18 is preferably more rigid than the conical part 17 and 14 of the respective third and first collars 16 and 13, thereby to enable the conical part 19 to form a stop means and thereby indicate that the tube 10 shall be inserted into the female urethra to an extent such that the conical part 19 comes into abutment with or essentially into abutment with that part of the female's body located in the region of the urethra outlet orifice. The radial extension of the third collar 18 will normally be such as to render it impossible in practice to tube the catheter tube 10 further than what is intended.

The aforedescribed catheter tube 10 can be readily handled by the user without the assistance of urologically trained personnel. When the tube 10 is inserted into the urethra of the female user, the conical part 14 of the first collar 13 will bend in the downstream direction, i.e. rearwardly towards the outlet opening 12, and slide against the urethra wall. Subsequent to inserting the catheter 10 to its optimal position, the catheter is withdrawn slightly, whereby the conical part 14 of the first collar 13 will attempt to bend in the opposite direction. The catheter tube is withdrawn only to a small extent sufficient for the conical part 14 to lift and take approximately the position illustration in Figure 1, wherewith the rounded edge of said part 14 will sealingly abut the muscular wall of the urethra while fixating the tube 10 in this position at the same time. Normal movement of the user, such as movement from a standing to a sitting or lying position, will not change the adopted position of the catheter tube 10 in the urethra. In the case of the aforesaid alternative embodiment in which the conical part 14 and/or the conical part 16 contains a fluid substance, such movement will cause said substance to permeate through the walls of said conical parts.

As will be seen from Figure 2, the inventive system is provided with known means 18' operative to prevent urine flowing out through the opening 12 and into the container. The reason why such means is provided will be explained in the following.

In the embodiment illustrated in Figure 2, the afore-described catheter tube 10 is fixedly or detachably connected to a flexible drainage hose 20, one end of which is connected in a known manner to the tube and which may be provided somewhere along its length with means for interrupting the flow of urine through the hose and thereby, for reasons already mentioned, therewith causing a build-up of urine in the bladder of the user. Such means may, for instance, have the form of a conventional clamp 21 (Figure 2a) which is clamped firmly around two hose parts bent back one upon the other. The closure means may also have the form of a known valve device, as indicated at 22 in Figures 2 and 2a.

The other end of the illustrated drainage hose 20 is connected to a urine collecting container, generally referenced 23, which is formed from a stretchable synthetic material. Thus, the container 23 will be flat, or essentially flat when not in use. The container 23 has an elongated configuration, with a smoothly rounded external shape. Connected to the container itself are two mutually opposing pieces 24, 25 provided with suspension slots 26, by means of which the container can be hung or hooked for instance, and a scale 26' for indicating the volume of liquid in the container.

The actual liquid accommodating part, referenced 23' in Figure 3, of the container 23 comprises a plurality of compartments 33 arranged within the container part 23'. These compartments 33 are in mutually spaced relationship, so as to form a plurality of liquid accommodating spaces or channels 33' which communicate with one another. This compartment arrangement ensures that the body liquid entering the container will be distributed uniformly between the various compartments or channels, whereby the container, even when full or partially full, will be easier to carry and less voluminous than conventional containers. The rounded corners of the container 23 also cause the pressure within the container to be distributed more uniformly while an arrangement in which the container lacks sharp edges will reduce the risk of damage to the container. Connected to the bottom of the container 23 is a short drainage pipe 27 which incorporates a known, manually operable valve means 29 in the vicinity of the outlet opening 28 of the container. This enables the container 23 to be emptied whenever desired.

The liquid accommodating part 23' of the container 23 may also advantageously be provided with an internally arranged reagent 30 of a known kind capable of being read externally of the container, so as to enable the possible occurrence of undesirable bacteria flora to be established.

Fitted to the upper end of the liquid collecting container 23 is a suitable non-return valve, for instance a check valve of the kind illustrated and described in my U.S. Patent Application No. 134,942. It will be obvious to those skilled in this art that any type of known valve which permits fluid to flow in solely one direction can be used. The improved container 23 is also provided with an overpressure valve indicated at 31. In its simplest form, this overpressure valve 31 may consist of a circular region formed integrally with the container material and consisting of a gas-permeable material, for instance material retailed under the registered trademark GOR-TEX. Gas present in the container 23 departs through the valve 31, thereby enabling the container to be filled to capacity, which has not been possible with hitherto known containers intended for this purpose.

It will be understood that the aforedescribed container 23 can be used for both male and female requirements, since it is solely the catheter tube which is configured particularly for insertion into the female urethra. Consequently, the container 23 can be combined with any other type of (known) catheter device or any other form of urine transfer device.

Although the specific object of the invention is to remedy, alleviate or guard against incontinence in women, it will be obvious to one skilled in this art that the improved system and the mutually coacting units or devices included in said system can replace highly advantageously other units and devices of present-day similar systems.

It also lies within the scope of the present invention to combine the catheter of said apparatus with a known urine collecting container, and also to combine novel the container of the described system with a known or conventional catheter.

## Claims

1. A system for the period or continuous drainage of urine from the bladder of females said system comprising firstly a urine catheter tube which includes an enlongated, hollow, flexible so-called catheter tube (10) intended for insertion into the urethra of a female patient, one open end of the catheter tube (10) communicating with the bladder and therewith functioning as an inlet opening for urine present in said bladder, and the other open end of which functions as an outlet opening for urine draining from the bladder, said catheter tube (10) including means for fixating said tube within the urethra, and secondly a container (23) which is connected to the outlet opening of the catheter tube either directly or indirectly and which is intended for collecting and temporarily storing urine draining from the bladder, said container (23) being provided in the region of said inlet with check valve means which permit urine to flow from the catheter tube (10) into the container (23) but prevent the flow of urine in the reverse direction, and the lower end of said container (23) being provided with means (28, 29) for emptying said container of its contents, **characterized** in that the means for fixating the catheter tube (10) in the urethra includes a first compliant collar (13) which is located on the outside of the tube in the region of the opening coacting with the bladder and which is concentrical with the longitudinal axis of the tube (10), said first collar being intended to sealingly abut the urethra wall, and further includes a second compliant collar (18) which is located adjacent to or in the vicinity of the region of the outlet opening of the catheter tube (10) such as to form means for restricting the extent to which the tube (10) is inserted into the urethra; and in that the urine collecting container (23) is divided into mutually communicating sections.

2. A system according to Claim 1, **charac****terized** in that the first and the second collars (13, 18) form a unit together with the catheter tube (10).

3. A system according to Claim 1 or 2, **char****acterized** in that the conical parts (14 and 19) of respective first and second collars (13 and 18) widen in mutually opposite directions.

4. A system according to one or more of the preceding claims, **characterized** in that the conical part (14) of the first collar (13) has a smaller radial extension than the conical part (19) of the second collar (18).

5. A system according to any one of the preceding claims, **characterized** by a third collar (16) having a conical part (17) which widens in the same direction as the conical part of the first collar (13) and having a radial extension which is smaller than the radial extension of the conical part (14) of the first collar (13); and in that the third collar (16) is located between the first and the second collars (13 and 18).

6. A system according to one or more of the preceding claims, **characterized** in that the conical parts (14 and 17) of the first (13) and/or the third (16) of said collars enclose a space for the accommodation of a liquid which, when the catheter tube (10) is in use, departs through permeable walls of the conical part or parts (14 or 17) when subjected to pressure.

7. A system according to any one of the preceding claims, **characterized** in that the conical part (19) of the second collar (18) solid.

8. A system according to any one of the preceding claims, **characterized** in that the catheter tube (10) has the same cross-sectional area throughout the whole of its length.

9. A system according to Claim 1, **charac****terized** by a drainage hose (20) which connects the catheter tube (10) to the urine collecting container (23).

10. A system according to Claim 9, **charac****terized** in that the drainage hose (20) and/or the catheter tube (10) is or are provided with means for preventing urine from flowing into the container (23).

11. A system according to Claim 1, **charac****terized** in that the container (23) is provided in the region of its upper part with valve means (31) operative to open at a predetermined overpressure created by urine present in the container and to restore the pressure balance in the container (23) (ambient pressure).

12. A system according to Claim 11, **charac****terized** in that said valve means (31) is located upstream of the check valve means.

13. A system according to Claim 1, **charac****terized** in that the container (23) is elongated and that division of the container into compartments (33) is effected essentially in the longitudinal direction of the container.

14. A system according to Claim 1, **charac****terized** in that the container (23) is elongated and in that division of the container into compartments (33) is effected essentially in the transverse direction of the container.

15. A system according to Claims 1, 13 or 14, **characterized** in that the compartments (33) in the container (23) are formed by narrow strips of material connected to mutually opposing inner walls of the container (23) and extending along and/or across the container; and in that each of said strips has a length which is at least half the length and/or the width of the container (23).

16. A system according to one or more of Claims 1, 13, 14 or 15, **characterized** in that the container (23) is provided with a reagent substance (30) which is visible externally of the container and operative to reveal the possible presence of bacteria flora.

17. A system according to one or more of the preceding claims, **characterized** in that the container (23) is provided with a scale (26') for indicating the volume of liquid present in the container.

## Patentansprüche

1. System zur periodischen oder kontinuierlichen Ableitung von Urin aus der Blase von Frauen, wobei dieses System umfaßt: erstens ein Urin-Katheterrohr, das ein längliches, hohles, flexibles sogenanntes Katheterrohr (10) aufweist, das zum Einführen in die Harnröhre eines weiblichen Patienten bestimmt ist, wobei ein offenes Ende des Katheterrohrs (10) mit der Blase in Verbindung steht und als Einlaßöffnung für den in der Blase enthaltenen Urin dient und dessen anderes offenes Ende als Auslaßöffnung für den aus der Blase abgeleiteten Urin dient, wobei das Katheterrohr (10) Mittel aufweist, um das Rohr in der Harnröhre festzulegen, und zweitens einen Behälter (23), der entweder unmittelbar oder mittelbar mit der Auslaßöffnung des Katheterrohrs verbunden ist und der dazu dient, den aus der Blase abgeleiteten Urin zu sammeln und vorübergehend zu speichern, wobei der Behälter (23) im Bereich des besagten Einlasses mit einer Rückschlagventileinrichtung versehen ist, die das Einströmen von Urin von dem Katheterrohr (10) in den Behälter (23) erlaubt, eine Strömung des Urins in der umgekehrten Richtung jedoch verhindert, und wobei das untere Ende des Behälters (23) mit Mitteln (28, 29) versehen ist, um den Inhalt des Behälters zu entleeren, dadurch **gekennzeichnet**, daß die Mittel zum Festlegen des Katheterrohrs (10) in der Harnröhre einen ersten schmiegsamen Kragen (13) umfassen, der an der Außenseite des Rohrs in dem Bereich der mit der Blase zusammenwirkenden Öffnung angeordnet und zur Längsachse des Rohrs (10) konzentrisch ist, wobei der erste Kragen dazu dient, an der Wand der Harnröhre dicht anzuliegen, und ferner einen zweiten schmiegsamen Kragen (18) umfaßt, der neben oder in der Nähe des Bereichs der Auslaßöffnung des Katheterrohrs (10) angeordnet ist, um Mittel zu bilden, die das Ausmaß beschränken, in dem das Rohr (10) in die Harnröhre eingeführt wird; und daß der Urinsammelbehälter (23) in miteinander in Verbindung stehende Abschnitte unterteilt ist.

2. System nach Anspruch 1, dadurch **gekennzeichnet,** daß der erste und der zweite Kragen (13, 18) zusammen mit dem Katheterrohr (10) eine Einheit bilden.

3. System nach Anspruch 1 oder 2, dadurch **gekennzeichnet,** daß die konischen Teile (14 und 19) des ersten und zweiten Kragens (13 und 18) sich in entgegengesetzten Richtungen erweitern.

4. System nach einem oder mehreren der vorhergehenden Ansprüche, dadurch **gekennzeichnet,** daß das konische Teil (14) des ersten Kragens (13) eine kleinere radiale Ausdehnung hat als das konische Teil (19) des zweiten Kragens (18).

5. System nach einem der vorhergehenden Ansprüche, **gekenn****zeichnet** durch einen dritten Kragen (16), der ein konisches Teil (17) hat, das sich in der gleichen Richtung wie das konische Teil des ersten Kragens (13) erweitert und das eine kleinere radiale Erstreckung hat als das konische Teil (14) des ersten Kragens (13); und daß der dritte Kragen (16) zwischen dem ersten und dem zweiten Kragen (13 und 18) angeordnet ist.

6. System nach einem oder mehreren der vorhergehenden Ansprüche, dadurch **gekennzeichnet,** daß die konischen Teile (14 und 16) des ersten und/oder des dritten Kragens (13 und/oder 16) einen Raum für die Aufnahme einer Flüssigkeit umschließen, die im Gebrauch des Katheterrohrs (10) durch durchlässige Wände des konischen Teils oder der Teile (14 oder 17) entweicht, wenn sie einem Druck ausgesetzt ist.

7. System nach einem der vorhergehenden Ansprüche, dadurch **gekennzeichnet,** daß das konische Teil (19) des zweiten Kragens (18) massiv ist.

8. System nach einem der vorhergehenden Ansprüche, dadurch **gekennzeichnet,** daß das Katheterrohr (10) über seine gesamte Länge die gleiche Querschnittsfläche hat.

9. System nach Anspruch 1, **gekennzeichnet** durch einen Ableitschlauch (20), der das Katheterrohr (10) mit dem Urinsammelbehälter (23) verbindet.

10. System nach Anspruch 9, dadurch **gekennzeichnet**, daß der Ableitschlauch (20) und/oder das Katheterrohr (10) mit Mitteln versehen ist bzw. sind, die den Urin daran hindern, in den Behälter (23) zu fließen.

11. System nach Anspruch 1, dadurch **gekennzeichnet,** daß der Behälter (23) im Bereich seines oberen Teils mit einer Ventileinrichtung (31) versehen ist, die bei einem von dem in dem Behälter enthaltenen Urin erzeugten bestimmten Überdruck öffnet, um in dem Behälter (23) einen Druckausgleich zu schaffen (Umgebungsdruck).

12. System nach Anspruch 11, dadurch **gekennzeichnet**, daß die Ventileinrichtung (31) stromaufwärts von der Rückschlagventileinrichtung angeordnet ist.

13. System nach Anspruch 1, dadurch **gekennzeichnet**, daß der Behälter (23) länglich ist und daß die Unterteilung des Behälters in Kammern (33) im wesentlichen in Längsrichtung des Behälters bewerkstelligt ist.

14. System nach Anspruch 1, dadurch **gekennzeichnet**, daß der Behälter (23) länglich ist und daß die Unterteilung des Behälters in Kammern (33) im wesentlichen in Querrichtung des Behälters bewerkstelligt ist.

15. System nach Anspruch 1, 13, oder 14, dadurch **gekenn****zeichnet,** daß die Kammern (33) in dem Behälter (23) von schmalen Materialstreifen gebildet sind, die mit gegenüberliegenden Innenwänden des Behälters (23) verbunden sind und sich in Längs- und/oder Querrichtung des Behälters erstrecken; und daß jeder dieser Streifen eine Länge hat, die mindestens der halben Länge und/oder Breite des Behälters (23) entspricht.

16. System nach einem oder mehreren der Ansprüche 1, 13, 14 oder 15, dadurch **gekennzeichnet,** daß der Behälter (23) mit einer Reaktionssubstanz (30) versehen ist, die von der Außenseite des Behälters sichtbar und geeignet ist, die mögliche Anwesenheit einer Bakterienflora anzuzeigen.

17. System nach einem oder mehreren der vorhergehenden Ansprüche, dadurch **gekennzeichnet,** daß der Behälter (23) mit einer Skala (26') versehen ist, um die in dem Behälter enthaltene Flüssigkeitsmenge anzuzeigen.

## Revendications

1. Système de drainage périodique ou continu de l'urine de la vessie d'une femme, ce système comprenant premièrement un tube à urine formant cathéter qui inclut ce que l'on appelle un tube cathéter (10) oblong creux et souple, destiné à être inséré dans l'urètre d'une patiente, une extrémité ouverte du tube cathéter (10) communiquant avec la vessie et servant d'ouverture d'entrée pour l'urine présente dans la vessie, tandis que son autre extrémité ouverte sert d'ouverture de sortie de l'urine drainée de la vessie, le tube cathéter (10) comprenant des moyens pour fixer ce tube dans l'urètre, et deuxièmement un récipient (23) qui communique avec l'ouverture de sortie du tube cathéter, soit directement, soit indirectement, et qui est destiné à recueillir et à stocker temporairement l'urine drainée de la vessie, ce récipient (23) étant muni, dans la région de l'entrée, d'un clapet antiretour qui permet à l'urine de s'écouler du tube cathéter (10) dans le récipient (23), mais qui empêche l'écoulement de l'urine dans le sens opposé, l'extrémité inférieure du récipient (23) étant munie de moyens (28, 29) destinés à vider ce récipient de son contenu, caractérisé en ce que les moyens de fixation du tube cathéter (10) dans l'urètre comprennent un premier collier (13) de conformation, qui est disposé à l'extérieur du tube dans la région de l'ouverture coopérant avec la vessie et qui est concentrique à l'axe longitudinal du tube (10), ce premier collier étant destiné à venir en butée d'une manière étanche sur la paroi de l'urètre et comprend, en outre, un deuxième collier (18) de conformation, qui est adjacent à la région de l'ouverture de sortie du tube cathéter (10) ou qui est à proximité de cette région, de manière à former des moyens de restriction de l'étendue suivant laquelle le tube (10) est inséré dans l'urètre; et en ce que le récipient (23) collecteur d'urine est subdivisé en des parties communiquant mutuellement.

2. Système suivant la revendication 1, caractérisé en ce que le premier et le deuxième colliers (13, 18) forment une unité ensemble avec le tube cathéter (10).

3. Système suivant la revendication 1 ou 2, caractérisé en ce que les parties coniques (14 et 19) des premier et deuxième colliers (13 et 18) s'élargissent en des directions mutuellement opposées.

4. Système suivant l'une ou plusieurs des revendications précédentes, caractérisé en ce que la partie (14) conique du premier collier (13) a une étendue radiale plus petite que la partie (19) conique du deuxième collier (18).

5. Système suivant l'une quelconque des revendications précédentes, caractérisé par un troisième collier (16) ayant une partie (17) conique qui s'élargit dans la même direction que la partie conique du premier collier (13) et ayant une étendue radiale qui est plus petite que l'étendue radiale de la partie (14) conique du premier collier (13) et en ce que le troisième collier (13) est interposé entre le premier et le deuxième colliers (13 et 18).

6. Système suivant l'une ou plusieurs des revendications précédentes, caractérisé en ce que les parties coniques (14 et 17) du premier (13) et/ou du troisième (16) colliers enferment un espace de réception d'un liquide qui, quand le tube cathéter (10) est en utilisation, passe à travers des parois perméables de la partie ou des parties (14 ou 17) coniques lorsqu'une pression est appliquée.

7. Système suivant l'une quelconque des revendications précédentes, caractérisé en ce que la partie (19) conique du deuxième collier (18) est pleine.

8. Système suivant l'une quelconque des revendications précédentes, caractérisé en ce que le tube cathéter (10) a la même section transversale sur toute sa longueur.

9. Système suivant la revendication 1, caractérisé par un tuyau (20) de drainage qui met le tube cathéter (10) en communication avec le récipient (23) de collecte d'urine.

10. Système suivant la revendication 9, caractérisé en ce que le tuyau (20) de drainage et/ou le tube cathéter (10) est ou sont munis de moyens destinés à empêcher de l'urine de s'écouler dans le récipient (23).

11. Système suivant la revendication 1, caractérisé en ce que le récipient (23) est muni, dans la région de sa partie supérieure, d'une soupape (31) qui s'ouvre à une surpression prescrite créée par de l'urine présente dans le récipient et qui restaure l'équilibre de pression dans le récipient (23) (pression ambiante).

12. Système suivant la revendication 11, caractérisé en ce que la vanne (31) est en amont du clapet antiretour.

13. Système suivant la revendication 1, caractérisé en ce que le récipient (23) est oblong et en ce que la division du récipient en compartiments (35) est effectuée essentiellement dans la direction longitudinale du récipient.

14. Système suivant la revendication 1, caractérisé en ce que le récipient (23) est oblong et en ce que la division du récipient en compartiments (33) est effectuée essentiellement dans la direction transversale du récipient.

15. Système suivant les revendications 1, 13 ou 14, caractérisé en ce que les compartiments (33) du récipient (23) sont formés de bandes étroites de matériau reliées à des parois intérieures mutuellement opposées du récipient (23) et s'étendant le long et/ou en travers du récipient; et en ce que chacune de ces bandes a une longueur qui est au moins égale à la moitié de la longueur et/ou de la largeur du récipient (23).

16. Système suivant l'une ou plusieurs des revendications 1, 13, 14 ou 15, caractérisé en ce que le récipient (23) est muni d'une substance (30) réactive, qui est visible de l'extérieur du récipient et qui est propre à révéler la présence possible de flore bactérienne.

17. Système suivant l'une ou plusieurs des revendications précédentes, caractérisé en ce que le récipient (23) est muni d'une graduation (26') indiquant le volume de liquide présent dans le récipient.
